(19) Europäisches Patentamt
European Patent Office
Office européen des brevets

(11) Veröffentlichungsnummer : **0 374 811 B1**

(12) EUROPÄISCHE PATENTSCHRIFT

(45) Veröffentlichungstag der Patentschrift : **03.03.93 Patentblatt 93/09**

(51) Int. Cl.$^5$ : **C07C 323/57**, C07C 317/48, A01N 37/50

(21) Anmeldenummer : **89123418.9**

(22) Anmeldetag : **19.12.89**

# (54) Schwefelhaltige Oximether und diese enthaltende Fungizide.

(30) Priorität : **23.12.88 DE 3843439**

(43) Veröffentlichungstag der Anmeldung : **27.06.90 Patentblatt 90/26**

(45) Bekanntmachung des Hinweises auf die Patenterteilung : **03.03.93 Patentblatt 93/09**

(84) Benannte Vertragsstaaten : **AT BE CH DE ES FR GB GR IT LI NL SE**

(56) Entgegenhaltungen :
**EP-A- 0 253 213**
**EP-A- 0 254 426**
**EP-A- 0 299 694**

(73) Patentinhaber : **BASF Aktiengesellschaft**
**Carl-Bosch-Strasse 38**
**W-6700 Ludwigshafen (DE)**

(72) Erfinder : **Wingert, Horst, Dr.**
**D 3,4**
**W-6800 Mannheim 1 (DE)**
Erfinder : **Brand, Siegbert, Dr.**
**Hegelstrasse 39**
**W-6940 Weinheim (DE)**
Erfinder : **Wenderoth, Bernd, Dr.**
**Schwalbenstrasse 26**
**W-6840 Lampertheim (DE)**
Erfinder : **Schütz, Franz, Dr.**
**Budapester Strasse 45**
**W-6700 Ludwigshafen (DE)**
Erfinder : **Sauter, Hubert, Dr.**
**Neckarpromenade 20**
**W-6800 Mannheim 1 (DE)**
Erfinder : **Röhl, Franz, Dr.**
**Karl-Otto-Braun-Strasse 8**
**W-6700 Ludwigshafen (DE)**
Erfinder : **Lorenz, Gisela, Dr.**
**Erlenweg 13**
**W-6730 Neustadt (DE)**
Erfinder : **Ammermann, Eberhard, Dr.**
**Sachsenstrasse 3**
**W-6700 Ludwigshafen (DE)**

**EP 0 374 811 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Oximether-Derivate, ihre Herstellung, diese enthaltende Fungizide und ihre Verwendung als Fungizide.

Es ist bekannt, Oximether wie zum Beispiel das 2-(Phenyloxymethyl)-phenyl-glyoxylsäuremethylester-O-methyloxim als Fungizide zu verwenden (EP 253 213, 254 426). Ihre fungizide Wirkung ist jedoch oft nicht ausreichend.

Es wurde nun gefunden, daß substituierte Oximether-Derivate der Formel

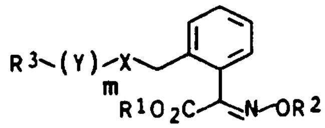

I,

in der

$R^1$, $R^2$ Wasserstoff oder $C_1$-$C_5$-Alkyl bedeutet,

X S, SO oder $SO_2$ bedeutet,

$R^3$ Wasserstoff, $C_3$-$C_8$-Cycloalkyl, Phenyl, Naphthyl, Chinolinyl oder Phenanthrenyl bedeutet, und diese Reste gegebenenfalls substituiert sind durch Halogen, Cyan, Nitro, Formyl, $C_1$-$C_{10}$-Alkyl, $C_1$-$C_4$-Alkoxy, $C_1$-$C_2$-Halogenalkyl, Aryl, $C_1$-$C_4$-Alkoxycarbonyl und

Y einen geradkettigen oder verzweigten Alkylen-, Alkenylen- oder Alkinylenrest bedeutet und

m die Zahlen 0 oder 1 bedeutet

eine ausgezeichnete fungizide Wirkung besitzen.

Die in der allgemeinen Formel I aufgeführten Reste können beispielsweise folgende Bedeutungen haben:

$R^1$ und $R^2$ sind gleich oder verschieden und stehen z. B. für Wasserstoff, $C_1$-$C_5$-Alkyl, Methyl, Ethyl, Propyl, i-Propyl, Butyl, Pentyl, Methyl wird bevorzugt.

X kann sein S, SO oder $SO_2$. S wird bevorzugt

$R^3$ kann z.B. Wasserstoff, $C_3$-$C_8$-Cycloalkyl (z. B. Cyclopropyl, Cyclobutyl, Cyclopentyl, Cyclohexyl, Cycloheptyl, Cyclooctyl), oder auch Phenyl, Naphthyl, Chinolinyl und Phenanthrenyl sein, wobei die Ringe gegebenenfalls durch einen bis zu drei gleichen oder verschiedenen der folgenden Reste substituiert sein können:

Halogen (z.B. Fluor, Chlor, Brom), Cyan, Nitro, Formyl, $C_1$-$C_{10}$-Alkyl (z.B. Methyl, Ethyl, n- oder iso-Propyl; n-, iso-, sec.- oder tert.-Butyl; n-, iso-, sec.-, tert.- oder neo-Pentyl; Hexyl, Heptyl, Octyl, Nonyl, Decyl), $C_1$-$C_2$-Halogenalkyl (z.B. Difluormethyl, Trifluormethyl, Chlormethyl, Dichlormethyl, Trichlormethyl, Pentafluorethyl), $C_1$-$C_4$-Alkoxy (z.B. Methoxy, Ethoxy, n- oder iso-Propoxy; n-, iso-, sec.- oder tert.-Butoxy), Aryl (z.B. Phenyl, Naphthyl, Pyridyl), $C_1$-$C_4$-Alkoxycarbonyl (z.B. Methoxycarbonyl, Ethoxycarbonyl, Propoxycarbonyl, Butoxycarbonyl).

Y kann sein z.B. $C_1$-$C_{10}$-Alkylen, Methylen, Ethylen, n- oder iso-Propylen; n-, iso-, sec.-oder tert.-Butylen; n-, iso-, sec.-, tert.- oder neo-Pentylen; Hexylen, Heptylen, Octylen, Nonylen, Decylen, $C_2$-$C_3$-Alkenylen, Vinylen, Allylen, Methylen wird bevorzugt,

m kann die Zahlen 0 oder 1 bedeuten.

Die neuen Verbindungen der Formel I können bei ihrer Herstellung aufgrund der C=N-Doppelbindung als E/Z-Isomerengemische anfallen, die in üblicher Weise, z.B. durch Kristallisation oder Chromatographie, in die einzelnen Komponenten getrennt werden können. Sowohl die einzelnen isomeren Verbindungen als auch ihre Gemische werden von der Erfindung umfaßt und sind als Fungizide brauchbar.

Die neuen Verbindungen der allgemeinen Formel I mit x = S lassen sich beispielsweise herstellen, indem man Thiole der Formel (II) mit einem Benzylhalogenid der allgemeinen Formel III umsetzt (Hal = Cl, Br, J).

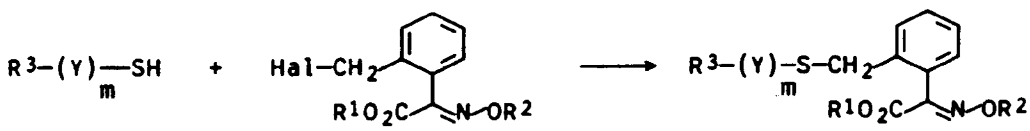

II III I

Die Umsetzungen zu den Verbindungen der Formel I können z.B. in einem inerten Lösungs- oder Verdünnungsmittel (z.B. Aceton, Acetonitril, Dimethylsulfoxid, Dioxan, Dimethylformamid, N-Methylpyrrolidon, N,N'-Dimethylpropylenharnstoff oder Pyridin) unter Verwendung einer Base (z.B. Natriumcarbonat, Kaliumcarbonat) durchgeführt werden. Außerdem kann es von Vorteil sein, der Reaktionsmischung einen Katalysator wie z.B. Tris-(3,6-dioxoheptyl)-amin zuzusetzen (J. Org. Chem. 50 (1985) 3717).

Alternativ kann auch so vorgegangen werden, daß man die Verbindungen der allgemeinen Formel II zunächst mit einer Base (z.B. Natriumhydroxid oder Kaliumhydroxid) in die entsprechenden Natrium-oder Kalisalze überführt und diese dann in einem inerten Lösungs- oder Verdünnungsmittel (z.B. Dimethylformamid) mit den Benzylhalogeniden der Formel III zu den entsprechenden Verbindungen der allgemeinen Formel I umsetzt.

Die Thiole der allgemeinen Formel $R^3$-$(Y)_m$-SH ($R^3$, Y und m haben die oben angegebene Bedeutung) sind entweder bekannt oder können durch Verfahren analog zu bekannten Verfahren hergestellt werden. Entsprechende Herstellverfahren sind z.B. beschrieben in Houben-Weyl, Methoden der organischen Chemie VI/3, 54 ff (1965).

Die ortho-substituierten Benzylhalogenide der Formel III lassen sich herstellen, indem man literaturbekannte α-Ketocarbonsäureester der Formel IV (vgl. z.B. J. M. Photis, Tetrahedron Lett. 1980, 3539) nach literaturbekannten Methoden an der Methylgruppe zu den Halogeniden der Formel V halogeniert.

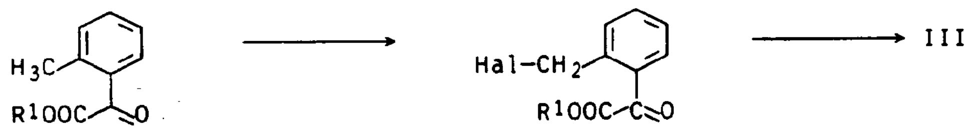
(IV) (V) III

Mit Brom oder Chlor in einem Lösungsmittel wie zum Beispiel Tetrachlormethan, gegebenenfalls unter Belichtung mit einer Lichtquelle (z.B. Hg-Dampf-Lampe, 300 W) oder mit N-Chlor- oder N-Bromsuccinimid (Horner, Winkelmann, Angew. Chem. 71, 349 (1959) gelangt man z.B. zu den α-Ketocarbonsäureestern der allgemeinen Formel V, wobei $R^1$ die obengenannten Bedeutungen hat.

Die Halogenide der Formel III lassen sich herstellen, indem man einen α-Ketocarbonsäureester der Formel V z.B. a) mit O-substituierten Hydroxylaminen der Formel $H_2N$-$OR^2$ umsetzt, in der $R^2$ die obengenannten Bedeutungen hat, oder b) mit Hydroxylamin zum entsprechenden Oxim und danach mit einem Alkylhalogenid der Formel $R^2$-Hal in der $R^2$ die obengenannten Bedeutungen hat und Hal ein Halogenatom (F, Cl, Br, J) bedeutet, oder mit einem Dialkylsulfat umsetzt.

Die neuen Verbindungen der allgemeinen Formel I (mit x = SO oder $SO_2$) erhält man durch Oxidation der entsprechenden Thioether der allgemeinen Formel I (mit X = S). Die Oxidation kann man z.B. mit Peroxoverbindungen wie z.B. Meta-Chlor-perbenzoesäure in einem inerten Lösungsmittel wie z.B. Toluol durchführen.

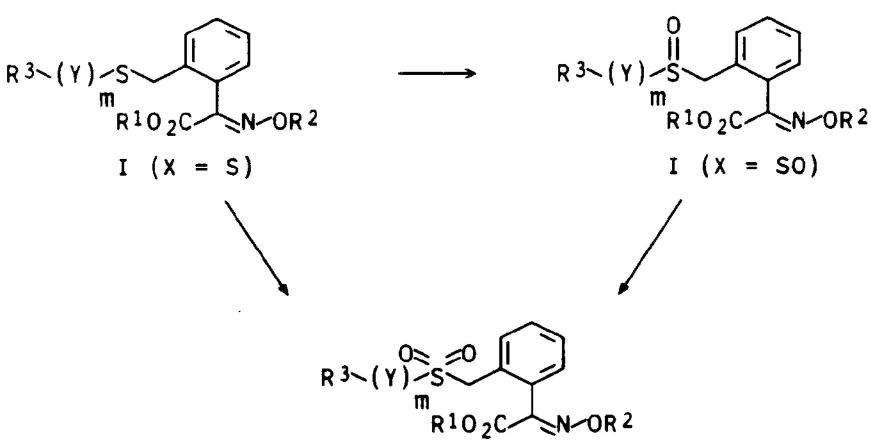
I (X = S) I (X = SO) I (X = $SO_2$)

(vgl. z.B. Houben-Weyl, Methoden der Org. Chemie IX, 213, 228 (1955)).

Die Halogenide der Formel III lassen sich auch herstellen, indem man einen α-Ketocarbonsäureester der Formel IV z.B. a) mit O-substituierten Hydroxylaminen der Formel $H_2N$-$OR^2$ umsetzt, in der $R^2$ die obengenannten Bedeutungen hat, oder b) mit Hydroxylamin zum entsprechenden Oxim und danach mit einem Alkylhalogenid der Formel $R^2$-Hal in der $R^2$ die obengenannten Bedeutungen hat und Hal ein Halogenatom (F, Cl, Br, J) bedeutet, oder mit einem Dialkylsulfat zu Oximether-Derivaten der allgemeinen Formel VI umsetzt, wobei $R^1$ und $R^2$ die jeweils oben genannten Bedeutungen haben.

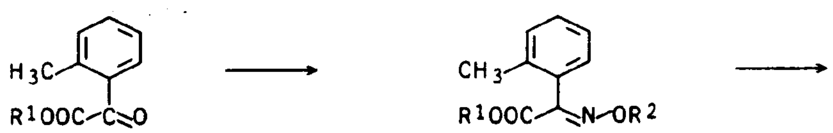

III

IV VI

Die Verbindungen der Formel VI können durch Halogenierung an der Methylgruppe nach literaturbekannten Methoden in die Halogenide der Formel III umgewandelt werden. Dies erreicht man zum Beispiel mit Brom oder Chlor in einem Lösungsmittel wie zum Beispiel Tetrachlormethan, gegebenenfalls unter Belichtung mit einer Lichtquelle (z.B. Hg-Dampf-Lampe, 300 W) oder mit N-Chlor- oder N-Bromsuccinimid (Horner, Winkelmann, Angew. Chem. 71, 349 (1959).

Die neuen Verbindungen der Formel I mit x = S können zum Beispiel auch in der Weise hergestellt werden, daß man die neuen α-Ketocarbonsäureester der Formel VII

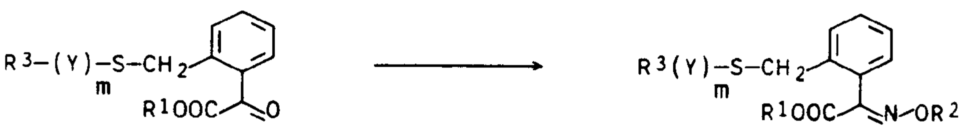

(VII) (I)

a) mit O-substituierten Hydroxylaminen der Formel $H_2NOR^2$ umsetzt, in der $R^2$ die obengenannten Bedeutungen hat, oder b) mit Hydroxylamin zum entsprechenden Oxim und danach mit einem Alkyhalogenid der Formel $R^2$-Hal, in der $R^2$ die obengenannten Bedeutungen hat und Hal ein Halogenatom (F, Cl, Br, J) bedeutet, oder mit einem Dialkylsulfat umsetzt.

Die neuen α-Ketocarbonsäureester der allgemeinen Formel VII sind wertvolle Zwischenprodukte. Sie können zum Beispiel hergestellt werden, indem man die obengenannte Verbindung der Formel V mit Thiolen der allgemeinen Formel II umsetzt.

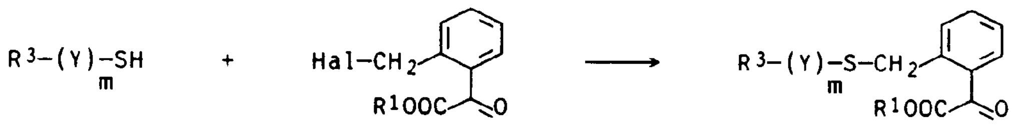

+

(II) (V) (VII)

Die Umsetzungen zu den Verbindungen der Formel VII können z.B. in einem inerten Lösungs- oder Verdünnungsmittel (z.B. Aceton, Acetonitril, Dimethylsulfoxid, Dioxan, Dimethylformamid, N-Methylpyrrolidon, N,N'-Dimethylpropylenharnstoff oder Pyridin) unter Verwendung einer Base (z.B. Natriumcarbonat, Kaliumcarbonat) durchgeführt werden. Außerdem kann es von Vorteil sein, der Reaktionsmischung einen Katalysator wie z.B. Tris-(3,6-dioxoheptyl)-amin zuzusetzen (J. Org. Chem. 50 (1985) 3717).

Alternativ kann auch so vorgegangen werden, daß man die Verbindungen der allgemeinen Formel II zunächst mit einer Base (z.B. Natriumhydroxid oder Kaliumhydroxid) in die entsprechenden Natrium-oder Kaliumsalze überführt und diese dann in einem inerten Lösungs-oder Verdünnungsmittel (z.B. Dimethylformamid) mit den Benzylhalogeniden der Formel V zu den neuen α-Ketocarbonsäureestern der allgemeinen Formel VII umsetzt.

Schwefelhaltige Oximether und diese enthaltende Fungizide

Beispiele

Die folgenden Beispiele sollen die Herstellung der neuen Wirkstoffe der allgemeinen Formel I erläutern.

Vorschrift 1

Herstellung von 2-(Brommethyl)-phenylglyoxylsäuremethylester

5,34 g (30 mmol) 2-Methylphenylglyoxylsäuremethylester und 5,34 g (30 mmol) N-Bromsuccinimid werden

in 1000 l Tetrachlormethan für eine Stunde mit einer 300 W-Hg-Dampf-Lampe bestrahlt. Dann wird die organische Phase 1x mit Wasser und 3x mit Natriumhydrogencarbonat-Lösung gewaschen und über Natriumsulfat/Natriumcarbonat getrocknet. Nach dem Einengen wird das Rohprodukt an Kieselgel mit Methyl-t.-Butylether/n-Hexan (1/9) chromatographiert. Man erhält 3,8 g (49 %) der obengenannten Verbindung als gelbes Öl.

$^1$H-NMR ($CDCl_3$):
$\delta$ = 3,97 (s, 3H); 4,90 (s, 2H); 7,4-7,8 (m, 4H).

IR (Film):
2955, 1740, 1689, 1435, 1318, 1207, 999 $cm^{-1}$

Vorschrift 2

Herstellung von 2-(Chlormethyl)-phenylglyoxylsäuremethylester

100 g (0,56 mol) 2-Methylphenylglyoxylsäuremethylester werden in 600 ml Tetrachlormethan vorgelegt. Dann wird unter Bestrahlung mit einer 300 W-Hg-Dampf-Lampe zum Rückfluß erhitzt und dabei 28 g (0,39 mol) Chlorgas eingeleitet. Nach ca. 50 % Umsatz (Kontrolle durch Dünnschichtchromatographie) wird die Reaktion abgebrochen und es wird eingeengt. Anschließend wird fraktioniert destilliert. Man erhält die Substanz als gelbes Öl.

$^1$H-NMR ($CDCl_3$):
$\delta$ = 3,97 (s, 3H); 5,0 (s, 2H); 7,4-7,8 (m, 4H).

Vorschrift 3

Herstellung von 2-(Brommethyl)-phenylglyoxylsäuremethylester-O-methyloxim

27,5 g (0, 133 mol) 2-Methyl-phenylglyoxylsäuremethylester-O-methyloxim gelöst in 400 ml Tetrachlormethan werden unter Rühren mit 21,4 g (0,133 mol) Brom versetzt. Dann wird unter Bestrahlung mit einer 300 W-Hg-Dampf-Lampe vier Stunden auf Rückflußtemperatur erhitzt. Danach wird eingeengt, in Essigester/Wasser aufgenommen, mit $H_2O$ gewaschen, mit Natriumsulfat getrocknet und eingeengt. Das Rohprodukt wird mit Cyclohexan/Essigester (9/1) an Kieselgel chromatographisch gereinigt. Es werden 17,4 g (46 %) der oben genannten Verbindung als Öl erhalten.

$^1$H-NMR ($CDCl_3$):
$\delta$ = 3,88 (s, 3H); 4,08 (s, 3H); 4,33 (s, 2H); 6,12-7,52 (m, 4H).

Vorschrift 4

Herstellung von 2-(Chlormethyl)-phenylglyoxylsäure-O-methyloxim

33,5 g (0,16 mol) 2-Methylphenylglyoxylsäuremethylester werden in 700 ml Chlorbenzol gelöst. Unter Bestrahlung mit einer 300 W-Hg-Dampf-Lampe werden innerhalb von einer Stunde 13,3 g Chlor eingegast. Die Innentemperatur wird dabei durch leichtes Heizen bei 75°C gehalten. Nach beendeter Reaktion wird der Kolbeninhalt 2 Stunden mit Stickstoff durchspült. Nach Entfernen des Lösungsmittels im Vakuum erhält man die Substanz als gelbes Öl.

$^1$H-NMR (DMSO):
$\delta$ = 3,75 (s, 3H); 4,00 (s, 3H); 4,55 (s, 2H); 7,05-7,45 ppm (m, 4H).

Beispiel 1

Herstellung von 2-(Phenylthiomethyl)-phenylglyoxylsäuremethylester-O-methyloxim (Verbindung 57 in Tabelle 1).

1,5 g (0,014 mol) Thiophenol und 4 g (0,014 mol) 2-(Brommethyl)-phenylglyoxylsäuremethylester-O-methyloxim (Vorschrift 3) werden in 100 ml Aceton gelöst. Man gibt 2,2 g Kaliumcarbonat und 0,1 g Kaliumjodid zu. Unter Rühren erhitzt man 17 Stunden unter Rückfluß. Nach dem Abkühlen wird mit 100 ml Wasser versetzt. Die Wasserphase wird mit Methylenchlorid extrahiert, man trocknet über Natriumsulfat und engt ein. Das Rohprodukt wird durch Verreiben mit n-Pentan zur Kristallisation gebracht. Man erhält 2,33 g (55 %) der oben genannten Verbindung als farblosen Feststoff (Fp = 60-65°C).

$^1$H-NMR ($CDCl_3$/TMS):

δ = 3,86 (s, 3H); 3,95 (s, 2H); 4,04 (s, 3H); 7,12-7,31 ppm (m, 9H).

Unter entsprechender Abwandlung der vorstehenden Angaben können die in der Tabelle 1 aufgeführten Verbindungen synthetisiert werden.

Tabelle 1

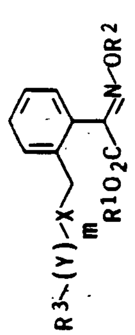

| Nr. | X | $(Y)_n$ | m | $R^3$ | $R^1$ | $R^2$ | IR ($cm^{-1}$) |
|---|---|---|---|---|---|---|---|
| 1 | S | $CH_2$ | 1 | H | $CH_3$ | $CH_3$ | |
| 2 | S | $CH_2$ | 1 | $CH_3$ | $CH_3$ | $CH_3$ | |
| 3 | S | $(CH_2)_2$ | 1 | $CH_3$ | $CH_3$ | $CH_3$ | |
| 4 | S | $(CH_2)_3$ | 1 | $CH_3$ | $CH_3$ | $CH_3$ | |
| 5 | S | $(CH_2)_4$ | 1 | $CH_3$ | $CH_3$ | $CH_3$ | |
| 6 | S | $(CH_2)_5$ | 1 | $CH_3$ | $CH_3$ | $CH_3$ | |
| 7 | S | $(CH_2)_6$ | 1 | $CH_3$ | $CH_3$ | $CH_3$ | |
| 8 | S | $(CH_2)_7$ | 1 | $CH_3$ | $CH_3$ | $CH_3$ | |
| 9 | S | $(CH_2)_8$ | 1 | $CH_3$ | $CH_3$ | $CH_3$ | |
| 10 | S | $(CH_2)_9$ | 1 | $CH_3$ | $CH_3$ | $CH_3$ | |
| 11 | S | CH(i-Pr) | 1 | $CH_3$ | $CH_3$ | $CH_3$ | |
| 12 | S | $CH(CH_3)$ | 1 | $CH_3$ | $CH_3$ | $CH_3$ | |
| 13 | S | CH(Et) | 1 | $CH_3$ | $CH_3$ | $CH_3$ | |
| 14 | S | CH(Pr) | 1 | $CH_3$ | $CH_3$ | $CH_3$ | |
| 15 | S | $CH_2$ | 1 | Cyclopropyl | $CH_3$ | $CH_3$ | |
| 16 | S | $CH_2$ | 1 | Cyclohexyl | $CH_3$ | $CH_3$ | |
| 17 | S | - | 0 | Cyclopentyl | $CH_3$ | $CH_3$ | |
| 18 | S | - | 0 | Cyclohexyl | $CH_3$ | $CH_3$ | |

Tabelle 1 (Fortsetzung)

| Nr. | X | $(Y)_n$ | m | $R^3$ | $R^1$ | $R^2$ | IR ($cm^{-1}$) |
|---|---|---|---|---|---|---|---|
| 19 | S | - | 0 | Cyclooctyl | $CH_3$ | $CH_3$ | |
| 20 | S | $CH_2$ | 1 | 1-Naphthyl | $CH_3$ | $CH_3$ | |
| 21 | S | $CH(CH_3)$ | 1 | Phenyl | $CH_3$ | $CH_3$ | |
| 22 | S | $CH_2$ | 1 | $CH=CH_2$ | $CH_3$ | $CH_3$ | |
| 23 | S | $CH_2$ | 1 | $C(CH_3)=CH_2$ | $CH_3$ | $CH_3$ | |
| 24 | S | $CH_2$ | 1 | 2-Chlor-phenyl | $CH_3$ | $CH_3$ | |
| 25 | S | $CH_2$ | 1 | 3-Chlor-phenyl | $CH_3$ | $CH_3$ | |
| 26 | S | $CH_2$ | 1 | 4-Chlor-phenyl | $CH_3$ | $CH_3$ | |
| 27 | S | $CH_2$ | 1 | 2-Brom-phenyl | $CH_3$ | $CH_3$ | |
| 28 | S | $CH_2$ | 1 | 3-Brom-phenyl | $CH_3$ | $CH_3$ | |
| 29 | S | $CH_2$ | 1 | 4-Brom-phenyl | $CH_3$ | $CH_3$ | |
| 30 | S | $CH_2$ | 1 | 2-Fluor-phenyl | $CH_3$ | $CH_3$ | |
| 31 | S | $CH_2$ | 1 | 3-Fluor-phenyl | $CH_3$ | $CH_3$ | |
| 32 | S | $CH_2$ | 1 | 4-Fluor-phenyl | $CH_3$ | $CH_3$ | |
| 33 | S | $CH_2$ | 1 | 2-Methyl-phenyl | $CH_3$ | $CH_3$ | |
| 34 | S | $CH_2$ | 1 | 3-Methyl-phenyl | $CH_3$ | $CH_3$ | |
| 35 | S | $CH_2$ | 1 | 4-Methyl-phenyl | $CH_3$ | $CH_3$ | |
| 36 | S | $CH_2$ | 1 | 4-Dodecyl-phenyl | $CH_3$ | $CH_3$ | |
| 37 | S | $CH_2$ | 1 | 2-Nitrophenyl | $CH_3$ | $CH_3$ | |
| 38 | S | $CH_2$ | 1 | 3-Nitrophenyl | $CH_3$ | $CH_3$ | |
| 39 | S | $CH_2$ | 1 | 4-Nitrophenyl | $CH_3$ | $CH_3$ | |
| 40 | S | $CH_2$ | 1 | 2-Methoxy-phenyl | $CH_3$ | $CH_3$ | |
| 41 | S | $CH_2$ | 1 | 3-Methoxy-phenyl | $CH_3$ | $CH_3$ | |
| 42 | S | $CH_2$ | 1 | 4-Methoxy-phenyl | $CH_3$ | $CH_3$ | |
| 43 | S | $CH_2$ | 1 | 2-Trifluormethyl-phenyl | $CH_3$ | $CH_3$ | |

Tabelle 1 (Fortsetzung)

| Nr. | X | $(Y)_n$ | m | $R^3$ | $R^1$ | $R^2$ | IR ($cm^{-1}$), Fp |
|---|---|---|---|---|---|---|---|
| 44 | S | $CH_2$ | 1 | 3-Trifluormethyl-phenyl | $CH_3$ | $CH_3$ | |
| 45 | S | $CH_2$ | 1 | 4-Trifluormethyl-phenyl | $CH_3$ | $CH_3$ | |
| 46 | S | $CH_2$ | 1 | 2,4-Dichlor-phenyl | $CH_3$ | $CH_3$ | |
| 47 | S | $CH_2$ | 1 | 3,4-Dichlor-phenyl | $CH_3$ | $CH_3$ | |
| 48 | S | $CH_2$ | 1 | 2-Chlor, 6-Fluor-phenyl | $CH_3$ | $CH_3$ | |
| 49 | S | $CH_2$ | 1 | 3,4-Dimethyl-phenyl | $CH_3$ | $CH_3$ | |
| 50 | S | $CH_2CH_2$ | 1 | Phenyl | $CH_3$ | $CH_3$ | |
| 51 | S | $CH_2CH_2CH_2$ | 1 | Phenyl | $CH_3$ | $CH_3$ | |
| 52 | S | $CH_2CH_2CH_2CH_2$ | 1 | 4-Chlorphenyl | $CH_3$ | $CH_3$ | |
| 53 | S | - | 0 | 1-Naphthyl | $CH_3$ | $CH_3$ | |
| 54 | S | - | 0 | 2-Naphthyl | $CH_3$ | $CH_3$ | 77-80°C |
| 55 | S | - | 0 | 3-Phenathrenyl | $CH_3$ | $CH_3$ | |
| 56 | S | - | 0 | 8-Chinolinyl | $CH_3$ | $CH_3$ | |
| 57 | S | - | 0 | Phenyl | $CH_3$ | $CH_3$ | 1727, 1480, 1438, 1321, 1218, 1967, 1045, 1018, 958, 743 |
| 58 | S | - | 0 | 2-Chlor-phenyl | $CH_3$ | $CH_3$ | 1740, 1452, 1431, 1307, 1219, 1066, 1047, 1008, 979, 743 |
| 59 | S | - | 0 | 3-Chlor-phenyl | $CH_3$ | $CH_3$ | 1727, 1580, 1467, 1217, 1088, 1061, 1010, 960, 777, 771 |
| 60 | S | - | 0 | 4-Chlor-phenyl | $CH_3$ | $CH_3$ | 82-85°C |
| 61 | S | - | 0 | 2-Brom-phenyl | $CH_3$ | $CH_3$ | |
| 62 | S | - | 0 | 3-Brom-phenyl | $CH_3$ | $CH_3$ | |
| 63 | S | - | 0 | 4-Brom-phenyl | $CH_3$ | $CH_3$ | |
| 64 | S | - | 0 | 2-Fluor-phenyl | $CH_3$ | $CH_3$ | |
| 65 | S | - | 0 | 3-Fluor-phenyl | $CH_3$ | $CH_3$ | |

Tabelle 1 (Fortsetzung)

| Nr. | X | $(Y)_n$ | m | $R^3$ | $R^1$ | $R^2$ | IR ($cm^{-1}$), Fp |
|---|---|---|---|---|---|---|---|
| 66 | S | - | 0 | 4-Fluor-phenyl | $CH_3$ | $CH_3$ | 1727, 1588, 1490, 1307, 1223, 1069, 1011, 982, 771, 761 |
| 67 | S | - | 0 | 2-Methyl-phenyl | $CH_3$ | $CH_3$ | 1735, 1448, 1433, 1297, 1226, 1066, 1008, 980, 763, 748 |
| 68 | S | - | 0 | 3-Methyl-phenyl | $CH_3$ | $CH_3$ | 39-40°C |
| 69 | S | - | 0 | 4-Methyl-phenyl | $CH_3$ | $CH_3$ | 68-71°C |
| 70 | S | - | 0 | 2-Ethyl-phenyl | $CH_3$ | $CH_3$ | |
| 71 | S | - | 0 | 3-Ethyl-phenyl | $CH_3$ | $CH_3$ | |
| 72 | S | - | 0 | 4-Ethyl-phenyl | $CH_3$ | $CH_3$ | |
| 73 | S | - | 0 | 2-iso-Propyl-phenyl | $CH_3$ | $CH_3$ | 53-55°C |
| 74 | S | - | 0 | 3-iso-Propyl-phenyl | $CH_3$ | $CH_3$ | |
| 75 | S | - | 0 | 4-iso-Propyl-phenyl | $CH_3$ | $CH_3$ | |
| 76 | S | - | 0 | 2-tert-Butyl-phenyl | $CH_3$ | $CH_3$ | |
| 77 | S | - | 0 | 3-tert-Butyl-phenyl | $CH_3$ | $CH_3$ | |
| 78 | S | - | 0 | 4-tert-Butyl-phenyl | $CH_3$ | $CH_3$ | 80-83°C |
| 79 | S | - | 0 | 4-Butyl-phenyl | $CH_3$ | $CH_3$ | |
| 80 | S | - | 0 | 4-Hexyl-phenyl | $CH_3$ | $CH_3$ | |
| 81 | S | - | 0 | 4-Nonyl-phenyl | $CH_3$ | $CH_3$ | |
| 82 | S | - | 0 | 4-Decyl-phenyl | $CH_3$ | $CH_3$ | |
| 83 | S | - | 0 | 2-Methoxy-phenyl | $CH_3$ | $CH_3$ | 1727, 1476, 1462, 1434, 1245, 1218, 1068, 1043, 1019, 753 |
| 84 | S | - | 0 | 3-Methoxy-phenyl | $CH_3$ | $CH_3$ | 60-64°C |
| 85 | S | - | 0 | 4-Methoxy-phenyl | $CH_3$ | $CH_3$ | 1727, 1591, 1494, 1439, 1298, 1285, 1246, 1217, 1067, 1018 |
| 86 | S | - | 0 | 2-Trifluormethyl-phenyl | $CH_3$ | $CH_3$ | |
| 87 | S | - | 0 | 3-Trifluormethyl-phenyl | $CH_3$ | $CH_3$ | 60-63°C |

Tabelle 1 (Fortsetzung)

| Nr. | X | $(Y)_n$ | m | $R^3$ | $R^1$ | $R^2$ | IR ($cm^{-1}$), Fp |
|---|---|---|---|---|---|---|---|
| 88 | S | - | 0 | 4-Trifluormethyl-phenyl | $CH_3$ | $CH_3$ | |
| 89 | S | - | 0 | 4-Formyl-phenyl | $CH_3$ | $CH_3$ | |
| 90 | S | - | 0 | 2-Nitro-phenyl | $CH_3$ | $CH_3$ | |
| 91 | S | - | 0 | 3-Nitro-phenyl | $CH_3$ | $CH_3$ | |
| 92 | S | - | 0 | 4-Nitro-phenyl | $CH_3$ | $CH_3$ | |
| 93 | S | - | 0 | 2,5-Dichlor-phenyl | $CH_3$ | $CH_3$ | 87-93°C |
| 94 | S | - | 0 | 2,6-Dichlor-phenyl | $CH_3$ | $CH_3$ | 113-117°C |
| 95 | S | - | 0 | 3,4-Dichlor-phenyl | $CH_3$ | $CH_3$ | |
| 96 | S | - | 0 | 2,3-Dichlor-phenyl | $CH_3$ | $CH_3$ | |
| 97 | S | - | 0 | 2,4-Dichlor-phenyl | $CH_3$ | $CH_3$ | |
| 98 | S | - | 0 | 3,5-Dichlor-phenyl | $CH_3$ | $CH_3$ | |
| 99 | S | - | 0 | 2,3,4-Trichlor-phenyl | $CH_3$ | $CH_3$ | 114-118°C |
| 100 | S | - | 0 | 2,4,5-Trichlor-phenyl | $CH_3$ | $CH_3$ | 121-124°C |
| 101 | S | - | 0 | 2,4,6-Trichlor-phenyl | $CH_3$ | $CH_3$ | |
| 102 | S | - | 0 | 2,3,4,6-Tetrachlor-phenyl | $CH_3$ | $CH_3$ | |
| 103 | S | - | 0 | 2,3,4,5,6-Pentachlor-phenyl | $CH_3$ | $CH_3$ | |
| 104 | S | - | 0 | 2,3,4,5-Tetrafluor-phenyl | $CH_3$ | $CH_3$ | |
| 105 | S | - | 0 | 2,3,5,6-Tetrafluor-phenyl | $CH_3$ | $CH_3$ | |
| 106 | S | - | 0 | 2,3,4,5,6-Pentafluor-phenyl | $CH_3$ | $CH_3$ | |
| 107 | S | - | 0 | 2-Chlor, 4-Fluor-phenyl | $CH_3$ | $CH_3$ | |
| 108 | S | - | 0 | 3-Chlor, 4-Fluor-phenyl | $CH_3$ | $CH_3$ | |
| 109 | S | - | 0 | 2-Chlor, 6-Methyl-phenyl | $CH_3$ | $CH_3$ | |
| 110 | S | - | 0 | 4-Chlor, 2-Methyl-phenyl | $CH_3$ | $CH_3$ | |
| 111 | S | - | 0 | 2,4-Dichlor, 5-Methyl-phenyl | $CH_3$ | $CH_3$ | |

Tabelle 1 (Fortsetzung)

| Nr. | X | $(Y)_n$ | m | $R^3$ | $R^1$ | $R^2$ | IR ($cm^{-1}$), Fp |
|---|---|---|---|---|---|---|---|
| 112 | S | - | 0 | 4-Chlor, 2,5-Dimethyl-phenyl | $CH_3$ | $CH_3$ | |
| 113 | S | - | 0 | 4-Brom, 3-Methyl-phenyl | $CH_3$ | $CH_3$ | |
| 114 | S | - | 0 | 3,5-Bistrifluormethyl-phenyl | $CH_3$ | $CH_3$ | |
| 115 | S | - | 0 | 2,5-Dimethyl-phenyl | $CH_3$ | $CH_3$ | |
| 116 | S | - | 0 | 2,4-Dimethyl-phenyl | $CH_3$ | $CH_3$ | |
| 117 | S | - | 0 | 2,5-Dimethyl-phenyl | $CH_3$ | $CH_3$ | |
| 118 | S | - | 0 | 2,6-Dimethyl-phenyl | $CH_3$ | $CH_3$ | |
| 119 | S | - | 0 | 3,4-Dimethyl-phenyl | $CH_3$ | $CH_3$ | |
| 120 | S | - | 0 | 3,5-Dimethyl-phenyl | $CH_3$ | $CH_3$ | |
| 121 | S | - | 0 | 2,4,5-Trimethyl-phenyl | $CH_3$ | $CH_3$ | |
| 122 | S | - | 0 | 2,6-Diethyl-phenyl | $CH_3$ | $CH_3$ | |
| 123 | S | - | 0 | 2,4-Di-tert.-Butyl-phenyl | $CH_3$ | $CH_3$ | |
| 124 | S | - | 0 | 2,5-Dimethoxy-phenyl | $CH_3$ | $CH_3$ | |
| 125 | S | - | 0 | 3,4-Dimethoxy-phenyl | $CH_3$ | $CH_3$ | |
| 126 | S | - | 0 | 2-Methyl, 4-tert.-Butyl-phenyl | $CH_3$ | $CH_3$ | 2963,2953,1728,1481,1437,1320 1217,1067,1045,1018 |
| 127 | S | - | 0 | 2-Methoxycarbonyl-phenyl | $CH_3$ | $CH_3$ | |
| 128 | S | - | 0 | 2-Ethoxycarbonyl-phenyl | $CH_3$ | $CH_3$ | |
| 129 | S | - | 0 | 2-Propoxycarbonyl-phenyl | $CH_3$ | $CH_3$ | |
| 130 | S | - | 0 | 2-Butoxycarbonyl-phenyl | $CH_3$ | $CH_3$ | |
| 131 | S | - | 0 | 2-Cyano-phenyl | $CH_3$ | $CH_3$ | |
| 132 | S | - | 0 | 3-Cyano-phenyl | $CH_3$ | $CH_3$ | |
| 133 | S | - | 0 | 4-Cyano-phenyl | $CH_3$ | $CH_3$ | |
| 134 | S | - | 0 | Phenyl | $CH_3$ | Et | |

Tabelle 1 (Fortsetzung)

| Nr. | X | $(Y)_n$ | m | $R^3$ | $R^1$ | $R^2$ | IR ($cm^{-1}$), Fp |
|---|---|---|---|---|---|---|---|
| 135 | S | - | 0 | Phenyl | Et | $CH_3$ | |
| 136 | S | - | 0 | Phenyl | $CH_3$ | n-Bu | |
| 137 | S | - | 0 | Phenyl | n-Bu | n-Bu | |

Tabelle 2

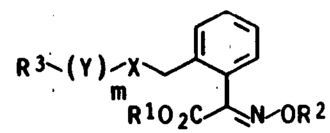

| Nr. | X | $(Y)_n$ | m | $R^3$ | $R^1$ | $R^2$ | IR ($cm^{-1}$) |
|---|---|---|---|---|---|---|---|
| 1 | SO | $CH_2$ | 1 | H | $CH_3$ | $CH_3$ | |
| 2 | SO | $CH_2$ | 1 | $CH_3$ | $CH_3$ | $CH_3$ | |
| 3 | SO | $(CH_2)_2$ | 1 | $CH_3$ | $CH_3$ | $CH_3$ | |
| 4 | SO | $(CH_2)_3$ | 1 | $CH_3$ | $CH_3$ | $CH_3$ | |
| 5 | SO | $(CH_2)_4$ | 1 | $CH_3$ | $CH_3$ | $CH_3$ | |
| 6 | SO | $(CH_2)_5$ | 1 | $CH_3$ | $CH_3$ | $CH_3$ | |
| 7 | SO | $(CH_2)_6$ | 1 | $CH_3$ | $CH_3$ | $CH_3$ | |
| 8 | SO | $(CH_2)_7$ | 1 | $CH_3$ | $CH_3$ | $CH_3$ | |
| 9 | SO | $(CH_2)_8$ | 1 | $CH_3$ | $CH_3$ | $CH_3$ | |
| 10 | SO | $(CH_2)_9$ | 1 | $CH_3$ | $CH_3$ | $CH_3$ | |
| 11 | SO | CH(i-Pr) | 1 | $CH_3$ | $CH_3$ | $CH_3$ | |
| 12 | SO | $CH(CH_3)$ | 1 | $CH_3$ | $CH_3$ | $CH_3$ | |
| 13 | SO | CH(Et) | 1 | $CH_3$ | $CH_3$ | $CH_3$ | |
| 14 | SO | CH(Pr) | 1 | $CH_3$ | $CH_3$ | $CH_3$ | |
| 15 | SO | $CH_2$ | 1 | Cyclopropyl | $CH_3$ | $CH_3$ | |
| 16 | SO | $CH_2$ | 1 | Cyclohexyl | $CH_3$ | $CH_3$ | |
| 17 | SO | - | 0 | Cyclopentyl | $CH_3$ | $CH_3$ | |
| 18 | SO | - | 0 | Cyclohexyl | $CH_3$ | $CH_3$ | |
| 19 | SO | - | 0 | Cyclooctyl | $CH_3$ | $CH_3$ | |

Tabelle 2 (Fortsetzung)

| Nr. | X | $(Y)_n$ | m | $R^3$ | $R^1$ | $R^2$ | IR ($cm^{-1}$) |
|---|---|---|---|---|---|---|---|
| 20 | SO | $CH_2$ | 1 | 1-Naphthyl | $CH_3$ | $CH_3$ | |
| 21 | SO | $CH(CH_3)$ | 1 | Phenyl | $CH_3$ | $CH_3$ | |
| 22 | SO | $CH_2$ | 1 | $CH=CH_2$ | $CH_3$ | $CH_3$ | |
| 23 | SO | $CH_2$ | 1 | $C(CH_3)=CH_2$ | $CH_3$ | $CH_3$ | |
| 24 | SO | $CH_2$ | 1 | 2-Chlor-phenyl | $CH_3$ | $CH_3$ | |
| 25 | SO | $CH_2$ | 1 | 3-Chlor-phenyl | $CH_3$ | $CH_3$ | |
| 26 | SO | $CH_2$ | 1 | 4-Chlor-phenyl | $CH_3$ | $CH_3$ | |
| 27 | SO | $CH_2$ | 1 | 2-Brom-phenyl | $CH_3$ | $CH_3$ | |
| 28 | SO | $CH_2$ | 1 | 3-Brom-phenyl | $CH_3$ | $CH_3$ | |
| 29 | SO | $CH_2$ | 1 | 4-Brom-phenyl | $CH_3$ | $CH_3$ | |
| 30 | SO | $CH_2$ | 1 | 2-Fluor-phenyl | $CH_3$ | $CH_3$ | |
| 31 | SO | $CH_2$ | 1 | 3-Fluor-phenyl | $CH_3$ | $CH_3$ | |
| 32 | SO | $CH_2$ | 1 | 4-Fluor-phenyl | $CH_3$ | $CH_3$ | |
| 33 | SO | $CH_2$ | 1 | 2-Methyl-phenyl | $CH_3$ | $CH_3$ | |
| 34 | SO | $CH_2$ | 1 | 3-Methyl-phenyl | $CH_3$ | $CH_3$ | |
| 35 | SO | $CH_2$ | 1 | 4-Methyl-phenyl | $CH_3$ | $CH_3$ | |
| 36 | SO | $CH_2$ | 1 | 4-Dodecyl-phenyl | $CH_3$ | $CH_3$ | |
| 37 | SO | $CH_2$ | 1 | 2-Nitrophenyl | $CH_3$ | $CH_3$ | |
| 38 | SO | $CH_2$ | 1 | 3-Nitrophenyl | $CH_3$ | $CH_3$ | |
| 39 | SO | $CH_2$ | 1 | 4-Nitrophenyl | $CH_3$ | $CH_3$ | |
| 40 | SO | $CH_2$ | 1 | 2-Methoxy-phenyl | $CH_3$ | $CH_3$ | |
| 41 | SO | $CH_2$ | 1 | 3-Methoxy-phenyl | $CH_3$ | $CH_3$ | |
| 42 | SO | $CH_2$ | 1 | 4-Methoxy-phenyl | $CH_3$ | $CH_3$ | |
| 43 | SO | $CH_2$ | 1 | 2-Trifluormethyl-phenyl | $CH_3$ | $CH_3$ | |
| 44 | SO | $CH_2$ | 1 | 3-Trifluormethyl-phenyl | $CH_3$ | $CH_3$ | |

Tabelle 2 (Fortsetzung)

| Nr. | X | $(Y)_n$ | m | $R^3$ | $R^1$ | $R^2$ | IR ($cm^{-1}$), Fp |
|---|---|---|---|---|---|---|---|
| 45 | SO | $CH_2$ | 1 | 4-Trifluormethyl-phenyl | $CH_3$ | $CH_3$ | |
| 46 | SO | $CH_2$ | 1 | 2,4-Dichlor-phenyl | $CH_3$ | $CH_3$ | |
| 47 | SO | $CH_2$ | 1 | 3,4-Dichlor-phenyl | $CH_3$ | $CH_3$ | |
| 48 | SO | $CH_2$ | 1 | 2-Chlor, 6-Fluor-phenyl | $CH_3$ | $CH_3$ | |
| 49 | SO | $CH_2$ | 1 | 3,4-Dimethyl-phenyl | $CH_3$ | $CH_3$ | |
| 50 | SO | $CH_2CH_2$ | 1 | Phenyl | $CH_3$ | $CH_3$ | |
| 51 | SO | $CH_2CH_2CH_2$ | 1 | Phenyl | $CH_3$ | $CH_3$ | |
| 52 | SO | $CH_2CH_2CH_2CH_2$ | 1 | 4-Chlorphenyl | $CH_3$ | $CH_3$ | |
| 53 | SO | - | 0 | 1-Naphthyl | $CH_3$ | $CH_3$ | |
| 54 | SO | - | 0 | 2-Naphthyl | $CH_3$ | $CH_3$ | |
| 55 | SO | - | 0 | 3-Phenandrenyl | $CH_3$ | $CH_3$ | |
| 56 | SO | - | 0 | 8-Chinolinyl | $CH_3$ | $CH_3$ | |
| 57 | SO | - | 0 | Phenyl | $CH_3$ | $CH_3$ | |
| 58 | SO | - | 0 | 2-Chlor-phenyl | $CH_3$ | $CH_3$ | 1729, 1461, 1438, 1323, 1217, 1169, 1067, 1047, 1017, 778 |
| 59 | SO | - | 0 | 3-Chlor-phenyl | $CH_3$ | $CH_3$ | |
| 60 | SO | - | 0 | 4-Chlor-phenyl | $CH_3$ | $CH_3$ | |
| 61 | SO | - | 0 | 2-Brom-phenyl | $CH_3$ | $CH_3$ | |
| 62 | SO | - | 0 | 3-Brom-phenyl | $CH_3$ | $CH_3$ | |
| 63 | SO | - | 0 | 4-Brom-phenyl | $CH_3$ | $CH_3$ | |
| 64 | SO | - | 0 | 2-Fluor-phenyl | $CH_3$ | $CH_3$ | |
| 65 | SO | - | 0 | 3-Fluor-phenyl | $CH_3$ | $CH_3$ | |
| 66 | SO | - | 0 | 4-Fluor-phenyl | $CH_3$ | $CH_3$ | |
| 67 | SO | - | 0 | 2-Methyl-phenyl | $CH_3$ | $CH_3$ | 110-115°C |
| 68 | SO | - | 0 | 3-Methyl-phenyl | $CH_3$ | $CH_3$ | |
| 69 | SO | - | 0 | 4-Methyl-phenyl | $CH_3$ | $CH_3$ | |

Tabelle 2 (Fortsetzung)

| Nr. | X | $(Y)_n$ | m | $R^3$ | $R^1$ | $R^2$ | IR ($cm^{-1}$) |
|---|---|---|---|---|---|---|---|
| 70 | SO | - | 0 | 2-Ethyl-phenyl | $CH_3$ | $CH_3$ | |
| 71 | SO | - | 0 | 3-Ethyl-phenyl | $CH_3$ | $CH_3$ | |
| 72 | SO | - | 0 | 4-Ethyl-phenyl | $CH_3$ | $CH_3$ | |
| 73 | SO | - | 0 | 2-iso-Propyl-phenyl | $CH_3$ | $CH_3$ | |
| 74 | SO | - | 0 | 3-iso-Propyl-phenyl | $CH_3$ | $CH_3$ | |
| 75 | SO | - | 0 | 4-iso-Propyl-phenyl | $CH_3$ | $CH_3$ | |
| 76 | SO | - | 0 | 2-tert-Butyl-phenyl | $CH_3$ | $CH_3$ | |
| 77 | SO | - | 0 | 3-tert-Butyl-phenyl | $CH_3$ | $CH_3$ | |
| 78 | SO | - | 0 | 4-tert-Butyl-phenyl | $CH_3$ | $CH_3$ | |
| 79 | SO | - | 0 | 4-Butyl-phenyl | $CH_3$ | $CH_3$ | |
| 80 | SO | - | 0 | 4-Hexyl-phenyl | $CH_3$ | $CH_3$ | |
| 81 | SO | - | 0 | 4-Nonyl-phenyl | $CH_3$ | $CH_3$ | |
| 82 | SO | - | 0 | 4-Decyl-phenyl | $CH_3$ | $CH_3$ | |
| 83 | SO | - | 0 | 2-Methoxy-phenyl | $CH_3$ | $CH_3$ | |
| 84 | SO | - | 0 | 3-Methoxy-phenyl | $CH_3$ | $CH_3$ | |
| 85 | SO | - | 0 | 4-Methoxy-phenyl | $CH_3$ | $CH_3$ | |
| 86 | SO | - | 0 | 2-Trifluormethyl-phenyl | $CH_3$ | $CH_3$ | |
| 87 | SO | - | 0 | 3-Trifluormethyl-phenyl | $CH_3$ | $CH_3$ | |
| 88 | SO | - | 0 | 4-Trifluormethyl-phenyl | $CH_3$ | $CH_3$ | |
| 89 | SO | - | 0 | 4-Formyl-phenyl | $CH_3$ | $CH_3$ | |
| 90 | SO | - | 0 | 2-Nitro-phenyl | $CH_3$ | $CH_3$ | |
| 91 | SO | - | 0 | 3-Nitro-phenyl | $CH_3$ | $CH_3$ | |
| 92 | SO | - | 0 | 4-Nitro-phenyl | $CH_3$ | $CH_3$ | |
| 93 | SO | - | 0 | 2,5-Dichlor-phenyl | $CH_3$ | $CH_3$ | |
| 94 | SO | - | 0 | 2,6-Dichlor-phenyl | $CH_3$ | $CH_3$ | |

Tabelle 2 (Fortsetzung)

| Nr. | X | $(Y)_n$ | m | $R^3$ | $R^1$ | $R^2$ | IR ($cm^{-1}$) |
|---|---|---|---|---|---|---|---|
| 95 | SO | - | 0 | 3,4-Dichlor-phenyl | $CH_3$ | $CH_3$ | |
| 96 | SO | - | 0 | 2,3-Dichlor-phenyl | $CH_3$ | $CH_3$ | |
| 97 | SO | - | 0 | 2,4-Dichlor-phenyl | $CH_3$ | $CH_3$ | |
| 98 | SO | - | 0 | 3,5-Dichlor-phenyl | $CH_3$ | $CH_3$ | |
| 99 | SO | - | 0 | 2,3,4-Trichlor-phenyl | $CH_3$ | $CH_3$ | |
| 100 | SO | - | 0 | 2,4,5-Trichlor-phenyl | $CH_3$ | $CH_3$ | |
| 101 | SO | - | 0 | 2,4,6-Trichlor-phenyl | $CH_3$ | $CH_3$ | |
| 102 | SO | - | 0 | 2,3,4,6-Tetrachlor-phenyl | $CH_3$ | $CH_3$ | |
| 103 | SO | - | 0 | 2,3,4,5,6-Pentachlor-phenyl | $CH_3$ | $CH_3$ | |
| 104 | SO | - | 0 | 2,3,4,5-Tetrafluor-phenyl | $CH_3$ | $CH_3$ | |
| 105 | SO | - | 0 | 2,3,5,6-Tetrafluor-phenyl | $CH_3$ | $CH_3$ | |
| 106 | SO | - | 0 | 2,3,4,5,6-Pentafluor-phenyl | $CH_3$ | $CH_3$ | |
| 107 | SO | - | 0 | 2-Chlor, 4-Fluor-phenyl | $CH_3$ | $CH_3$ | |
| 108 | SO | - | 0 | 3-Chlor, 4-Fluor-phenyl | $CH_3$ | $CH_3$ | |
| 109 | SO | - | 0 | 2-Chlor, 6-Methyl-phenyl | $CH_3$ | $CH_3$ | |
| 110 | SO | - | 0 | 4-Chlor, 2-Methyl-phenyl | $CH_3$ | $CH_3$ | |
| 111 | SO | - | 0 | 2,4-Dichlor, 5-Methyl-phenyl | $CH_3$ | $CH_3$ | |
| 112 | SO | - | 0 | 4-Chlor, 2,5-Dimethyl-phenyl | $CH_3$ | $CH_3$ | |
| 113 | SO | - | 0 | 4-Brom, 3-Methyl-phenyl | $CH_3$ | $CH_3$ | |
| 114 | SO | - | 0 | 3,5-Bistrifluormethyl-phenyl | $CH_3$ | $CH_3$ | |
| 115 | SO | - | 0 | 2,5-Dimethyl-phenyl | $CH_3$ | $CH_3$ | |
| 116 | SO | - | 0 | 2,4-Dimethyl-phenyl | $CH_3$ | $CH_3$ | |
| 117 | SO | - | 0 | 2,5-Dimethyl-phenyl | $CH_3$ | $CH_3$ | |
| 118 | SO | - | 0 | 2,6-Dimethyl-phenyl | $CH_3$ | $CH_3$ | |
| 119 | SO | - | 0 | 3,4-Dimethyl-phenyl | $CH_3$ | $CH_3$ | |

Tabelle 2 (Fortsetzung)

| Nr. | X | $(Y)_n$ | m | $R^3$ | $R^1$ | $R^2$ | IR ($cm^{-1}$) |
|---|---|---|---|---|---|---|---|
| 120 | SO | - | 0 | 3,5-Dimethyl-phenyl | $CH_3$ | $CH_3$ | |
| 121 | SO | - | 0 | 2,4,5-Trimethyl-phenyl | $CH_3$ | $CH_3$ | |
| 122 | SO | - | 0 | 2,6-Diethyl-phenyl | $CH_3$ | $CH_3$ | |
| 123 | SO | - | 0 | 2,4-Di-tert.-Butyl-phenyl | $CH_3$ | $CH_3$ | |
| 124 | SO | - | 0 | 2,5-Dimethoxy-phenyl | $CH_3$ | $CH_3$ | |
| 125 | SO | - | 0 | 3,4-Dimethoxy-phenyl | $CH_3$ | $CH_3$ | |
| 126 | SO | - | 0 | 2-Methyl, 4-tert.-Butyl-phenyl | $CH_3$ | $CH_3$ | |
| 127 | SO | - | 0 | 2-Methoxycarbonyl-phenyl | $CH_3$ | $CH_3$ | |
| 128 | SO | - | 0 | 2-Ethoxycarbonyl-phenyl | $CH_3$ | $CH_3$ | |
| 129 | SO | - | 0 | 2-Propoxycarbonyl-phenyl | $CH_3$ | $CH_3$ | |
| 130 | SO | - | 0 | 2-Butoxycarbonyl-phenyl | $CH_3$ | $CH_3$ | |
| 131 | SO | - | 0 | 2-Cyano-phenyl | $CH_3$ | $CH_3$ | |
| 132 | SO | - | 0 | 3-Cyano-phenyl | $CH_3$ | $CH_3$ | |
| 133 | SO | - | 0 | 4-Cyano-phenyl | $CH_3$ | $CH_3$ | |
| 134 | SO | - | 0 | Phenyl | $CH_3$ | Et | |
| 135 | SO | - | 0 | Phenyl | Et | $CH_3$ | |
| 136 | SO | - | 0 | Phenyl | $CH_3$ | n-Bu | |
| 137 | SO | - | 0 | Phenyl | n-Bu | n-Bu | |

Tabelle 3

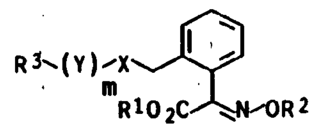

| Nr. | X | (Y)$_n$ | n | $R^3$ | $R^1$ | $R^2$ | IR ($cm^{-1}$) |
|---|---|---|---|---|---|---|---|
| 1 | $SO_2$ | $CH_2$ | 1 | H | $CH_3$ | $CH_3$ | |
| 2 | $SO_2$ | $CH_2$ | 1 | $CH_3$ | $CH_3$ | $CH_3$ | |
| 3 | $SO_2$ | $(CH_2)_2$ | 1 | $CH_3$ | $CH_3$ | $CH_3$ | |
| 4 | $SO_2$ | $(CH_2)_3$ | 1 | $CH_3$ | $CH_3$ | $CH_3$ | |
| 5 | $SO_2$ | $(CH_2)_4$ | 1 | $CH_3$ | $CH_3$ | $CH_3$ | |
| 6 | $SO_2$ | $(CH_2)_5$ | 1 | $CH_3$ | $CH_3$ | $CH_3$ | |
| 7 | $SO_2$ | $(CH_2)_6$ | 1 | $CH_3$ | $CH_3$ | $CH_3$ | |
| 8 | $SO_2$ | $(CH_2)_7$ | 1 | $CH_3$ | $CH_3$ | $CH_3$ | |
| 9 | $SO_2$ | $(CH_2)_8$ | 1 | $CH_3$ | $CH_3$ | $CH_3$ | |
| 10 | $SO_2$ | $(CH_2)_9$ | 1 | $CH_3$ | $CH_3$ | $CH_3$ | |
| 11 | $SO_2$ | CH(i-Pr) | 1 | $CH_3$ | $CH_3$ | $CH_3$ | |
| 12 | $SO_2$ | $CH(CH_3)$ | 1 | $CH_3$ | $CH_3$ | $CH_3$ | |
| 13 | $SO_2$ | CH(Et) | 1 | $CH_3$ | $CH_3$ | $CH_3$ | |
| 14 | $SO_2$ | CH(Pr) | 1 | $CH_3$ | $CH_3$ | $CH_3$ | |
| 15 | $SO_2$ | $CH_2$ | 1 | Cyclopropyl | $CH_3$ | $CH_3$ | |
| 16 | $SO_2$ | $CH_2$ | 1 | Cyclohexyl | $CH_3$ | $CH_3$ | |
| 17 | $SO_2$ | - | 0 | Cyclopentyl | $CH_3$ | $CH_3$ | |
| 18 | $SO_2$ | - | 0 | Cyclohexyl | $CH_3$ | $CH_3$ | |
| 19 | $SO_2$ | - | 0 | Cyclooctyl | $CH_3$ | $CH_3$ | |

Tabelle 3 (Fortsetzung)

| Nr. | X | $(Y)_n$ | n | $R^3$ | $R^1$ | $R^2$ | IR ($cm^{-1}$) |
|---|---|---|---|---|---|---|---|
| 20 | $SO_2$ | $CH_2$ | 1 | 1-Naphthyl | $CH_3$ | $CH_3$ | |
| 21 | $SO_2$ | $CH(CH_3)$ | 1 | Phenyl | $CH_3$ | $CH_3$ | |
| 22 | $SO_2$ | $CH_2$ | 1 | $CH{=}CH_2$ | $CH_3$ | $CH_3$ | |
| 23 | $SO_2$ | $CH_2$ | 1 | $C(CH_3){=}CH_2$ | $CH_3$ | $CH_3$ | |
| 24 | $SO_2$ | $CH_2$ | 1 | 2-Chlor-phenyl | $CH_3$ | $CH_3$ | |
| 25 | $SO_2$ | $CH_2$ | 1 | 3-Chlor-phenyl | $CH_3$ | $CH_3$ | |
| 26 | $SO_2$ | $CH_2$ | 1 | 4-Chlor-phenyl | $CH_3$ | $CH_3$ | |
| 27 | $SO_2$ | $CH_2$ | 1 | 2-Brom-phenyl | $CH_3$ | $CH_3$ | |
| 28 | $SO_2$ | $CH_2$ | 1 | 3-Brom-phenyl | $CH_3$ | $CH_3$ | |
| 29 | $SO_2$ | $CH_2$ | 1 | 4-Brom-phenyl | $CH_3$ | $CH_3$ | |
| 30 | $SO_2$ | $CH_2$ | 1 | 2-Fluor-phenyl | $CH_3$ | $CH_3$ | |
| 31 | $SO_2$ | $CH_2$ | 1 | 3-Fluor-phenyl | $CH_3$ | $CH_3$ | |
| 32 | $SO_2$ | $CH_2$ | 1 | 4-Fluor-phenyl | $CH_3$ | $CH_3$ | |
| 33 | $SO_2$ | $CH_2$ | 1 | 2-Methyl-phenyl | $CH_3$ | $CH_3$ | |
| 34 | $SO_2$ | $CH_2$ | 1 | 3-Methyl-phenyl | $CH_3$ | $CH_3$ | |
| 35 | $SO_2$ | $CH_2$ | 1 | 4-Methyl-phenyl | $CH_3$ | $CH_3$ | |
| 36 | $SO_2$ | $CH_2$ | 1 | 4-Dodecyl-phenyl | $CH_3$ | $CH_3$ | |
| 37 | $SO_2$ | $CH_2$ | 1 | 2-Nitrophenyl | $CH_3$ | $CH_3$ | |
| 38 | $SO_2$ | $CH_2$ | 1 | 3-Nitrophenyl | $CH_3$ | $CH_3$ | |
| 39 | $SO_2$ | $CH_2$ | 1 | 4-Nitrophenyl | $CH_3$ | $CH_3$ | |
| 40 | $SO_2$ | $CH_2$ | 1 | 2-Methoxy-phenyl | $CH_3$ | $CH_3$ | |
| 41 | $SO_2$ | $CH_2$ | 1 | 3-Methoxy-phenyl | $CH_3$ | $CH_3$ | |
| 42 | $SO_2$ | $CH_2$ | 1 | 4-Methoxy-phenyl | $CH_3$ | $CH_3$ | |
| 43 | $SO_2$ | $CH_2$ | 1 | 2-Trifluormethyl-phenyl | $CH_3$ | $CH_3$ | |
| 44 | $SO_2$ | $CH_2$ | 1 | 3-Trifluormethyl-phenyl | $CH_3$ | $CH_3$ | |

Tabelle 3 (Fortsetzung)

| Nr. | X | $(Y)_n$ | n | $R^3$ | $R^1$ | $R^2$ | IR ($cm^{-1}$), Fp |
|---|---|---|---|---|---|---|---|
| 45 | $SO_2$ | $CH_2$ | 1 | 4-Trifluormethyl-phenyl | $CH_3$ | $CH_3$ | |
| 46 | $SO_2$ | $CH_2$ | 1 | 2,4-Dichlor-phenyl | $CH_3$ | $CH_3$ | |
| 47 | $SO_2$ | $CH_2$ | 1 | 3,4-Dichlor-phenyl | $CH_3$ | $CH_3$ | |
| 48 | $SO_2$ | $CH_2$ | 1 | 2-Chlor, 6-Fluor-phenyl | $CH_3$ | $CH_3$ | |
| 49 | $SO_2$ | $CH_2$ | 1 | 3,4-Dimethyl-phenyl | $CH_3$ | $CH_3$ | |
| 50 | $SO_2$ | $CH_2CH_2$ | 1 | Phenyl | $CH_3$ | $CH_3$ | |
| 51 | $SO_2$ | $CH_2CH_2CH_2$ | 1 | Phenyl | $CH_3$ | $CH_3$ | |
| 52 | $SO_2$ | $CH_2CH_2CH_2CH_2$ | 1 | 4-Chlorphenyl | $CH_3$ | $CH_3$ | |
| 53 | $SO_2$ | - | 0 | 1-Naphthyl | $CH_3$ | $CH_3$ | |
| 54 | $SO_2$ | - | 0 | 2-Naphthyl | $CH_3$ | $CH_3$ | |
| 55 | $SO_2$ | - | 0 | 3-Phenandrenyl | $CH_3$ | $CH_3$ | |
| 56 | $SO_2$ | - | 0 | 8-Chinolinyl | $CH_3$ | $CH_3$ | |
| 57 | $SO_2$ | - | 0 | Phenyl | $CH_3$ | $CH_3$ | |
| 58 | $SO_2$ | - | 0 | 2-Chlor-phenyl | $CH_3$ | $CH_3$ | 143-150°C |
| 59 | $SO_2$ | - | 0 | 3-Chlor-phenyl | $CH_3$ | $CH_3$ | 126-130°C |
| 60 | $SO_2$ | - | 0 | 4-Chlor-phenyl | $CH_3$ | $CH_3$ | |
| 61 | $SO_2$ | - | 0 | 2-Brom-phenyl | $CH_3$ | $CH_3$ | |
| 62 | $SO_2$ | - | 0 | 3-Brom-phenyl | $CH_3$ | $CH_3$ | |
| 63 | $SO_2$ | - | 0 | 4-Brom-phenyl | $CH_3$ | $CH_3$ | |
| 64 | $SO_2$ | - | 0 | 2-Fluor-phenyl | $CH_3$ | $CH_3$ | |
| 65 | $SO_2$ | - | 0 | 3-Fluor-phenyl | $CH_3$ | $CH_3$ | |
| 66 | $SO_2$ | - | 0 | 4-Fluor-phenyl | $CH_3$ | $CH_3$ | |
| 67 | $SO_2$ | - | 0 | 2-Methyl-phenyl | $CH_3$ | $CH_3$ | 124-127°C |
| 68 | $SO_2$ | - | 0 | 3-Methyl-phenyl | $CH_3$ | $CH_3$ | |
| 69 | $SO_2$ | - | 0 | 4-Methyl-phenyl | $CH_3$ | $CH_3$ | |

Tabelle 3 (Fortsetzung)

| Nr. | X | $(Y)_n$ | n | $R^3$ | $R^1$ | $R^2$ | IR ($cm^{-1}$) |
|---|---|---|---|---|---|---|---|
| 70 | $SO_2$ | - | 0 | 2-Ethyl-phenyl | $CH_3$ | $CH_3$ | |
| 71 | $SO_2$ | - | 0 | 3-Ethyl-phenyl | $CH_3$ | $CH_3$ | |
| 72 | $SO_2$ | - | 0 | 4-Ethyl-phenyl | $CH_3$ | $CH_3$ | |
| 73 | $SO_2$ | - | 0 | 2-iso-Propyl-phenyl | $CH_3$ | $CH_3$ | |
| 74 | $SO_2$ | - | 0 | 3-iso-Propyl-phenyl | $CH_3$ | $CH_3$ | |
| 75 | $SO_2$ | - | 0 | 4-iso-Propyl-phenyl | $CH_3$ | $CH_3$ | |
| 76 | $SO_2$ | - | 0 | 2-tert-Butyl-phenyl | $CH_3$ | $CH_3$ | |
| 77 | $SO_2$ | - | 0 | 3-tert-Butyl-phenyl | $CH_3$ | $CH_3$ | |
| 78 | $SO_2$ | - | 0 | 4-tert-Butyl-phenyl | $CH_3$ | $CH_3$ | |
| 79 | $SO_2$ | - | 0 | 4-Butyl-phenyl | $CH_3$ | $CH_3$ | |
| 80 | $SO_2$ | - | 0 | 4-Hexyl-phenyl | $CH_3$ | $CH_3$ | |
| 81 | $SO_2$ | - | 0 | 4-Nonyl-phenyl | $CH_3$ | $CH_3$ | |
| 82 | $SO_2$ | - | 0 | 4-Decyl-phenyl | $CH_3$ | $CH_3$ | |
| 83 | $SO_2$ | - | 0 | 2-Methoxy-phenyl | $CH_3$ | $CH_3$ | |
| 84 | $SO_2$ | - | 0 | 3-Methoxy-phenyl | $CH_3$ | $CH_3$ | |
| 85 | $SO_2$ | - | 0 | 4-Methoxy-phenyl | $CH_3$ | $CH_3$ | |
| 86 | $SO_2$ | - | 0 | 2-Trifluormethyl-phenyl | $CH_3$ | $CH_3$ | |
| 87 | $SO_2$ | - | 0 | 3-Trifluormethyl-phenyl | $CH_3$ | $CH_3$ | |
| 88 | $SO_2$ | - | 0 | 4-Trifluormethyl-phenyl | $CH_3$ | $CH_3$ | |
| 89 | $SO_2$ | - | 0 | 4-Formyl-phenyl | $CH_3$ | $CH_3$ | |
| 90 | $SO_2$ | - | 0 | 2-Nitro-phenyl | $CH_3$ | $CH_3$ | |
| 91 | $SO_2$ | - | 0 | 3-Nitro-phenyl | $CH_3$ | $CH_3$ | |
| 92 | $SO_2$ | - | 0 | 4-Nitro-phenyl | $CH_3$ | $CH_3$ | |
| 93 | $SO_2$ | - | 0 | 2,5-Dichlor-phenyl | $CH_3$ | $CH_3$ | |
| 94 | $SO_2$ | - | 0 | 2,6-Dichlor-phenyl | $CH_3$ | $CH_3$ | |

Tabelle 3 (Fortsetzung)

| Nr. | X | (Y)$_n$ | n | $R^3$ | $R^1$ | $R^2$ | IR ($cm^{-1}$) |
|---|---|---|---|---|---|---|---|
| 95 | $SO_2$ | - | 0 | 3,4-Dichlor-phenyl | $CH_3$ | $CH_3$ | |
| 96 | $SO_2$ | - | 0 | 2,3-Dichlor-phenyl | $CH_3$ | $CH_3$ | |
| 97 | $SO_2$ | - | 0 | 2,4-Dichlor-phenyl | $CH_3$ | $CH_3$ | |
| 98 | $SO_2$ | - | 0 | 3,5-Dichlor-phenyl | $CH_3$ | $CH_3$ | |
| 99 | $SO_2$ | - | 0 | 2,3,4-Trichlor-phenyl | $CH_3$ | $CH_3$ | |
| 100 | $SO_2$ | - | 0 | 2,4,5-Trichlor-phenyl | $CH_3$ | $CH_3$ | |
| 101 | $SO_2$ | - | 0 | 2,4,6-Trichlor-phenyl | $CH_3$ | $CH_3$ | |
| 102 | $SO_2$ | - | 0 | 2,3,4,6-Tetrachlor-phenyl | $CH_3$ | $CH_3$ | |
| 103 | $SO_2$ | - | 0 | 2,3,4,5,6-Pentachlor-phenyl | $CH_3$ | $CH_3$ | |
| 104 | $SO_2$ | - | 0 | 2,3,4,5-Tetrafluor-phenyl | $CH_3$ | $CH_3$ | |
| 105 | $SO_2$ | - | 0 | 2,3,5,6-Tetrafluor-phenyl | $CH_3$ | $CH_3$ | |
| 106 | $SO_2$ | - | 0 | 2,3,4,5,6-Pentafluor-phenyl | $CH_3$ | $CH_3$ | |
| 107 | $SO_2$ | - | 0 | 2-Chlor, 4-Fluor-phenyl | $CH_3$ | $CH_3$ | |
| 108 | $SO_2$ | - | 0 | 3-Chlor, 4-Fluor-phenyl | $CH_3$ | $CH_3$ | |
| 109 | $SO_2$ | - | 0 | 2-Chlor, 6-Methyl-phenyl | $CH_3$ | $CH_3$ | |
| 110 | $SO_2$ | - | 0 | 4-Chlor, 2-Methyl-phenyl | $CH_3$ | $CH_3$ | |
| 111 | $SO_2$ | - | 0 | 2,4-Dichlor, 5-Methyl-phenyl | $CH_3$ | $CH_3$ | |
| 112 | $SO_2$ | - | 0 | 4-Chlor, 2,5-Dimethyl-phenyl | $CH_3$ | $CH_3$ | |
| 113 | $SO_2$ | - | 0 | 4-Brom, 3-Methyl-phenyl | $CH_3$ | $CH_3$ | |
| 114 | $SO_2$ | - | 0 | 3,5-Bistrifluormethyl-phenyl | $CH_3$ | $CH_3$ | |
| 115 | $SO_2$ | - | 0 | 2,5-Dimethyl-phenyl | $CH_3$ | $CH_3$ | |
| 116 | $SO_2$ | - | 0 | 2,4-Dimethyl-phenyl | $CH_3$ | $CH_3$ | |
| 117 | $SO_2$ | - | 0 | 2,5-Dimethyl-phenyl | $CH_3$ | $CH_3$ | |
| 118 | $SO_2$ | - | 0 | 2,6-Dimethyl-phenyl | $CH_3$ | $CH_3$ | |
| 119 | $SO_2$ | - | 0 | 3,4-Dimethyl-phenyl | $CH_3$ | $CH_3$ | |

Tabelle 3 (Fortsetzung)

| Nr. | X | $(Y)_n$ | n | $R^3$ | $R^1$ | $R^2$ | IR ($cm^{-1}$) |
|---|---|---|---|---|---|---|---|
| 120 | $SO_2$ | - | 0 | 3,5-Dimethyl-phenyl | $CH_3$ | $CH_3$ | |
| 121 | $SO_2$ | - | 0 | 2,4,5-Trimethyl-phenyl | $CH_3$ | $CH_3$ | |
| 122 | $SO_2$ | - | 0 | 2,6-Diethyl-phenyl | $CH_3$ | $CH_3$ | |
| 123 | $SO_2$ | - | 0 | 2,4-Di-tert.-Butyl-phenyl | $CH_3$ | $CH_3$ | |
| 124 | $SO_2$ | - | 0 | 2,5-Dimethoxy-phenyl | $CH_3$ | $CH_3$ | |
| 125 | $SO_2$ | - | 0 | 3,4-Dimethoxy-phenyl | $CH_3$ | $CH_3$ | |
| 126 | $SO_2$ | - | 0 | 2-Methyl, 4-tert.-Butyl-phenyl | $CH_3$ | $CH_3$ | |
| 127 | $SO_2$ | - | 0 | 2-Methoxycarbonyl-phenyl | $CH_3$ | $CH_3$ | |
| 128 | $SO_2$ | - | 0 | 2-Ethoxycarbonyl-phenyl | $CH_3$ | $CH_3$ | |
| 129 | $SO_2$ | - | 0 | 2-Propoxycarbonyl-phenyl | $CH_3$ | $CH_3$ | |
| 130 | $SO_2$ | - | 0 | 2-Butoxycarbonyl-phenyl | $CH_3$ | $CH_3$ | |
| 131 | $SO_2$ | - | 0 | 2-Cyano-phenyl | $CH_3$ | $CH_3$ | |
| 132 | $SO_2$ | - | 0 | 3-Cyano-phenyl | $CH_3$ | $CH_3$ | |
| 133 | $SO_2$ | - | 0 | 4-Cyano-phenyl | $CH_3$ | $CH_3$ | |
| 134 | $SO_2$ | - | 0 | Phenyl | $CH_3$ | Et | |
| 135 | $SO_2$ | - | 0 | Phenyl | Et | $CH_3$ | |
| 136 | $SO_2$ | - | 0 | Phenyl | Me | n-Bu | |
| 137 | $SO_2$ | - | 0 | Phenyl | n-Bu | n-Bu | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,

Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 57 mit 10 Gew.-Teilen N-methyl $\alpha$-pyrrolidon und erhält eine Lösung, die zur Anwendung in Form kleinster Tropfen geeignet ist.

II. 20 Gew.-Teile der Verbindung Nr. 58 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 59 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 67 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 57 werden mit 3 Gew.-Teilen des Natriumsalzes der Diisobutylnaph-

thalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 58 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 59 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 67 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 57 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des Fungiziden Wirkungsspektrums

Anwendungsbeispiele

Als Vergleichswirkstoff wurde 2-(Phenyloxymethyl)-phenylglyoxalsäure-O-methyloxim (A) - bekannt aus EP 253 213 - benutzt.

Anwendungsbeispiel

Wirksamkeit gegen Pyrenophora teres

Gerstenkeimlinge der Sorte "Igri" wurden im Zweiblattstadium mit wäßrigen Suspensionen, die 80 % ( Gew.%) Wirkstoff und 20 % Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in eine Klimakammer mit hoher Luftfeuchtigkeit bei 18°C gestellt. Anschließend wurden die Pflanzen im Gewächshaus bei 20 bis 22°C und 70 % relativer Luftfeuchtigkeit für weitere 5 Tage kultiviert. Dann wurde das Ausmaß der Symptomentwicklung ermittelt.

Das Ergebnis zeigt, daß die Wirkstoffe 57, 58, 59 und 67 bei der Anwendung als 0,05 %ige (Gew.%) Spritzbrühe eine bessere fungizide Wirkung zeigen (100 %) als der bekannte Vergleichswirkstoff A (65 %).

## Patentansprüche

1. Oximether-Derivate der Formel

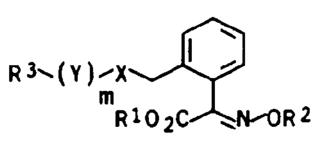

I

in der

$R^1$, $R^2$ Wasserstoff oder $C_1$-$C_5$-Alkyl bedeutet,

X S, SO oder $SO_2$ bedeutet,

$R^3$ Wasserstoff, $C_3$-$C_8$-Cycloalkyl, Phenyl, Naphthyl, Chinolinyl oder Phenanthrenyl bedeutet, und diese Reste gegebenenfalls substituiert sind durch Halogen, Cyan, Nitro, Formyl, $C_1$-$C_{10}$-Alkyl, $C_1$-$C_4$-Alkoxy, $C_1$-$C_2$-Halogenalkyl, Aryl, $C_1$-$C_4$-Alkoxycarbonyl und

Y einen geradkettigen oder verzweigten Alkylen-, Alkenylen-oder Alkinylenrest bedeutet und

m die Zahlen 0 oder 1 bedeutet.

2. Verfahren zur Herstellung von Oximether-Derivaten der Formel I (mit X = S) gemäß Anspruch 1, dadurch gekennzeichnet, daß man ein Thiol der allgemeinen Formel

$$R^3\text{-}(Y)_m\text{-}SH \qquad (II)$$

in der $R^3$, Y und m die obengenannte Bedeutung haben, mit einem Benzylhalogenid der allgemeinen Formel III umsetzt,

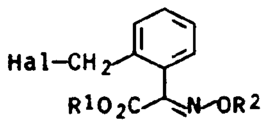

(III)

in der Hal für Brom, Chlor oder Jod steht und in der $R^1$ und $R^2$ die obengenannte Bedeutung hat und gegebenenfalls das so erhaltene Oximetherderivat der Formel I (mit X = S) zu einem Oximetherderivat der Formel I (mit X = SO, $SO_2$) umsetzt.

3. Verfahren zur Bekämpfung von Pilzen, dadurch gekennzeichnet, daß man die Pilze oder die von Pilzbefall bedrohten Materialien, Pflanzen, Saatgüter oder den Erdboden mit einer fungizid wirksamen Menge eines Oximetherderivats der Formel I behandelt

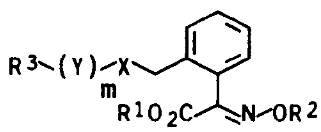

in der

$R^1$, $R^2$ Wasserstoff oder $C_1$-$C_5$-Alkyl bedeutet,

X S, SO oder $SO_2$ bedeutet,

$R^3$ Wasserstoff, $C_3$-$C_8$-Cycloalkyl, Phenyl, Naphthyl, Chinolinyl oder Phenanthrenyl bedeutet, und diese Reste gegebenenfalls substituiert sind durch Halogen, Cyan, Nitro, Formyl, $C_1$-$C_{10}$-Alkyl, $C_1$-$C_4$-Alkoxy, $C_1$-$C_2$-Halogenalkyl, Aryl, $C_1$-$C_4$-Alkoxycarbonyl und

Y einen geradkettigen oder verzweigten Alkylen-, Alkenylen-oder Alkinylenrest bedeutet und

m die Zahlen 0 oder 1 bedeutet.

4. Fungizid, enthaltend einen inerten Trägerstoff und eine fungizid wirksame Menge eines Oximetherderivats der Formel I

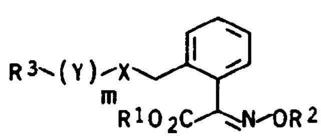

in der

$R^1$, $R^2$ Wasserstoff oder $C_1$-$C_5$-Alkyl bedeutet,

X S, SO oder $SO_2$ bedeutet,

$R^3$ Wasserstoff, $C_3$-$C_8$-Cycloalkyl, Phenyl, Naphthyl, Chinolinyl oder Phenanthrenyl bedeutet, und diese Reste gegebenenfalls substituiert sind durch Halogen, Cyan, Nitro, Formyl, $C_1$-$C_{10}$-Alkyl, $C_1$-$C_4$-Alkoxy, $C_1$-$C_2$-Halogenalkyl, Aryl, $C_1$-$C_4$-Alkoxycarbonyl und

Y einen geradkettigen oder verzweigten Alkylen-, Alkenylen- oder Alkinylenrest bedeutet und

m die Zahlen 0 oder 1 bedeutet.

5. Verfahren zur Herstellung eines fungiziden Mittels, dadurch gekennzeichnet, daß man eine oder mehrere Verbindungen der Formel I gemäß Anspruch 1 mit einem festen oder flüssigen Trägerstoff sowie gegebenenfalls mit einem oder mehreren oberflächenaktiven Mitteln mischt.

6. Verbindung gemäß Anspruch 1, dadurch gekennzeichnet, daß $R^1$ und $R^2$ Methyl, X den Rest S, $R^3$ Phenyl

und $Y_m$ Methylen bedeutet.

7. Verbindung gemäß Anspruch 1, dadurch gekennzeichnet, daß $R^1$ und $R^2$ Methyl, X den Rest S, $R^3$ 2-Chlorphenyl und $Y_m$ Methylen bedeutet.

8. Verbindung gemäß Anspruch 1, dadurch gekennzeichnet, daß $R^1$ und $R^2$ Methyl, X den Rest S, $R^3$ 3-Chlorphenyl und $Y_m$ Methylen bedeutet.

9. Verbindung gemäß Anspruch 1, dadurch gekennzeichnet, daß $R^1$ und $R^2$ Methyl, X den Rest S, $R^3$ 2-Methylphenyl und $Y_m$ Methylen bedeutet.

10. 2-(Chormethyl)-phenylglyoxylsäure-O-methyloxim.

## Claims

1. An oxime ether derivative of the formula

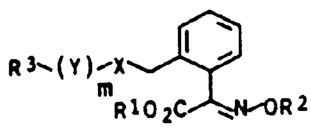

I

where $R^1$ and $R^2$ are each hydrogen or $C_1$-$C_5$-alkyl, X is S, SO or $SO_2$, $R_3$ is hydrogen, $C_3$-$C_8$-cycloalkyl, phenyl, naphthyl, quinolinyl of phenanthrenyl, these radicals being unsubstituted or substituted by halogen, cyano, nitro, formyl, $C_1$-$C_{10}$-alkyl, $C_1$-$C_4$-alkoxy, $C_1$-$C_2$-haloalkyl, aryl or $C_1$-$C_4$-alkoxycarbonyl, Y is a straight-chain or branched alkylene, alkenylene or alkynylene radical, and m is 0 or 1.

2. A process for preparing oxime ether derivatives of the formula I (with X=S) as claimed in claim 1, which comprises reacting a thiol of the formula

$$R^3\text{-}(Y)_m\text{-SH} \qquad II$$

where $R^3$, Y and m have the abovementioned meanings, with a benzyl halide of the formula III

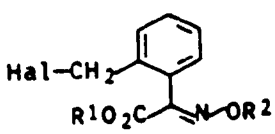

III

where HAl is bromine, chlorine or iodine, and where $R^1$ and $R^2$ have the abovementioned meanings, and, if required, converting the resulting oxime ether derivative of the formula I (with X=S) into an oxime ether derivative of the formula I (with X=S0, $SO_2$).

3. A method for controlling fungi, wherein the fungi, or the materials, plants, seed or soil threatened by fungus attack are treated with a fungicidally effective amount of an oxime ether derivative of the formula I

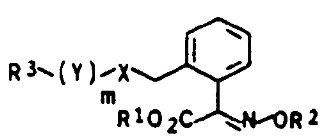

where $R^1$ and $R^2$ are each hydrogen or $C_1$-$C_5$-alkyl, X is S, SO or $SO_2$, $R^3$ is hydrogen, $C_3$-$C_8$-cycloalkyl, phenyl, naphthyl, quinolinyl or phenanthrenyl, these radicals being unsubstituted or substituted by halogen, cyano, nitro, formyl, $C_1$-$C_{10}$-alkyl, $C_1$-$C_4$-alkoxy, $C_1$-$C_2$-haloalkyl, aryl or $C_1$-$C_4$-alkoxycarbonyl, Y is a straight-chain or branched alkylene, alkenylene or alkynylene radical, and m is 0 or 1.

4. A fungicide containing an inert carrier and a fungicidally effective amount of an oxime ether derivative of the formula I

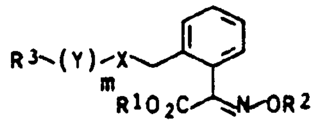

where $R^1$ and $R^2$ are each hydrogen or $C_1$-$C_5$-alkyl, X is S, SO or $SO_2$, $R^3$ is hydrogen, $C_3$-$C_8$-cycloalkyl, phenyl, naphthyl, quinolinyl or phenanthrenyl, these radicals being unsubstituted or substituted by halogen, cyano, nitro, formyl, $C_1$-$C_{10}$-alkyl, $C_1$-$C_4$-alkoxy, $C_1$-$C_2$-haloalkyl, aryl, or $C_1$-$C_4$-alkoxycarbonyl, Y is a straight-chain or branched alkylene, alkenylene or alkynylene radical, and m is 0 or 1.

5. A process for producing a fungicidal composition, which comprises mixing one or more compounds of the formula I as claimed in claim 1, with A solid or liquid carrier and, if required, with one or more surfactants.

6. A compound as claimed in claim 1, wherein $R^1$ and $R^2$ are methyl, X is S, $R^3$ is phenyl and $Y_m$ is methylene.

7. A compound as claimed in claim 1, wherein $R^1$ and $R^2$ are methyl, X is S, $R^3$ is 2-chlorophenyl and $Y_m$ is methylene.

8. A compound as claimed in claim 1, wherein $R^1$ and $R^2$ are methyl, X is S, $R^3$ is 3-chlorophenyl and $Y_m$ is methylene.

9. A compound as claimed in claim 1, wherein $R^1$ and $R^2$ are methyl, X is S, $R^3$ is 2-methylphenyl and $Y_m$ is methylene.

10. 2-(Chloromethyl)-phenylglyoxylic acid-O-methyl-oxime.

## Revendications

1. Dérivés d'éthers d'oximes de formule

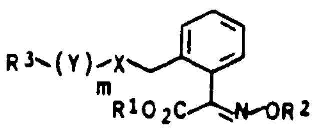

I

dans laquelle

$R^1$, $R^2$ représentent chacun l'hydrogène ou un groupe alkyle en $C_1$-$C_5$,

X représente S, SO ou $SO_2$,

$R^3$ représente l'hydrogène, un groupe cycloalkyle en $C_3$-$C_8$, phényle, naphtyle, quinoléinyle ou phénanthrénylé, ces groupes pouvant éventuellement être substitués par des halogènes, des groupes cyano, nitro, formyle, alkyle en $C_1$-$C_{10}$, alcoxy en $C_1$-$C_4$, halogénoalkyle en $C_1$-$C_2$, aryle, (alcoxy en $C_1$-$C_4$)-carbonyle, et

Y représente un groupe alkylène, alcénylène ou alcynylène à chaîne droite ou ramifiée, et

m est égal à 0 ou 1.

2. Procédé de préparation des dérivés d'éthers d'oximes de formule I (dans laquelle X = S) de la revendication 1, caractérisé en ce que l'on fait réagir un thiol de formule générale

$$R^3\text{-}(Y)_m\text{-}SH \quad (II)$$

dans laquelle $R^3$, Y et m ont les significations indiquées ci-dessus, avec un halogénure de benzyle de formule générale III

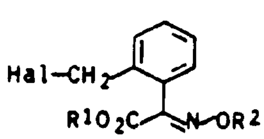

(III)

dans laquelle Hal représente le brome, le chlore ou l'iode et $R^1$ et $R^2$ ont les significations indiquées ci-dessus, et le cas échéant on convertit le dérivé d'éther d'oxime de formule I (dans laquelle X = S) ainsi obtenu en un dérivé d'éther d'oxime de formule I (dans laquelle X = SO, $SO_2$).

**3.** Procédé pour combattre les mycètes, caractérisé en ce que l'on traite les mycètes ou les matières, plantes, semences ou sols menacés d'une attaque par les mycètes par une quantité fongicide efficace d'un dérivé d'éther d'oxime de formule I.

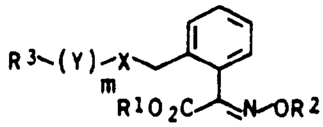

dans laquelle

$R^1$, $R^2$ représentent chacun l'hydrogène ou un groupe alkyle en C1-C5,

X représente S, SO ou $SO_2$,

$R^3$ représente l'hydrogène, un groupe cycloalkyle en C3-C8, phényle, naphtyle, quinoléinyle ou phénanthrénylé, et ces groupes peuvent éventuellement être eux-mêmes substitués par des halogènes, des groupes cyano, nitro, formyle, alkyle en C1-C10, alcoxy en C1-C4, halogénoalkyle en C1-C2, aryle, (alcoxy en C1-C4)-carbonyle, et

Y représente un groupe alkylène, alcénylène ou alcynylène à chaîne droite ou ramifiée, et

m est égal à 0 ou 1.

**4.** Produit fongicide contenant un véhicule inerte et une quantité fongicide efficace d'un dérivé d'éther d'oxime de formule I

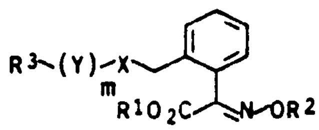

dans laquelle

$R^1$, $R^2$ représentent chacun l'hydrogène ou un groupe alkyle en C1-C5,

X représente S, SO ou $SO_2$,

$R^3$ représente l'hydrogène, un groupe cycloalkyle en C3-C8, phényle, naphtyle, quinoléinyle ou phénanthrénylé, ces groupes peuvent eux-mêmes être éventuellement substitués par des halogènes, des groupes cyano, nitro, formyle, alkyle en C1-C10, alcoxy en C1-C4, halogénoalkyle en C1-C2, aryle, (alcoxy en C1-C4)-carbonyle, et

Y représente un groupe alkylène, alcénylène ou alcynylène à chaîne droite ou ramifiée, et

m est égal à 0 ou 1.

**5.** Procédé de préparation d'un produit fongicide, caractérisé en ce que l'on mélange un ou plusieurs composés de formule I de la revendication 1 avec un véhicule solide ou liquide et le cas échéant un ou plusieurs agents tensioactifs.

**6.** Composé selon la revendication 1, caractérisé en ce que $R^1$ et $R^2$ représentent des groupes méthyle, X représente S, $R^3$ représente un groupe phényle et $Y_m$ un groupe méthylène.

**7.** Composé selon la revendication 1, caractérisé en ce que $R^1$ et $R^2$ représentent des groupes méthyle, X représente S, $R^3$ représente un groupe 2-chlorophényle et $Y_m$ représente un groupe méthylène.

**8.** Composé selon la revendication 1, caractérisé en ce que $R^1$ et $R^2$ représentent des groupes méthyle, X représente S, $R^3$ un groupe 3-chlorophényle et $Y_m$ un groupe méthylène.

**9.** Composé selon la revendication 1, caractérisé en ce que R1 et $R^2$ représentent des groupes méthyle, X représente S, $R^3$ représente un groupe 2-méthylphényle et $Y_m$ un groupe méthylène.

**10.** L'O-méthyloxime de l'acide 2-(chlorométhyl)-phénylglyoxylique.